# FASCICULE DE BREVET EUROPEEN

(11) **EP 0 998 464 B1**
(45) Date de publication et mention de la délivrance du brevet: **15.10.2003**
(21) Numéro de dépôt: 98939674.2
(22) Date de dépôt: 15.07.1998
(51) Int. Cl.: C07D 241/12, A61K 31/495

(54) **DERIVES DE POLYHYDROXYALKYLPYRAZINES, LEUR PREPARATION ET LES MEDICAMENTS LES CONTENANT**
POLYHYDROXYALKYLPYRAZINE DERIVATE,DEREN HERSTELLUNG UND DIESE ENTHALTENDE ARZNEIMITTELN
POLYHYDROXYALKYLPYRAZINE DERIVATIVES, THEIR PREPARATION AND MEDICINES CONTAINING THEM

(30) Priorité: 17.07.1997 FR 9709056; 21.07.1997 FR 9709206
(43) Date de publication de la demande: 10.05.2000
(73) Titulaire: Aventis Pharma S.A., 92160 Antony (FR)
(72) Inventeur: BASHIARDES, Georges, F-94320 Thiais (FR); CARRY, Jean-Christophe, F-92190 Meudon (FR); EVERS, Michel, F-94510 La Queue en Brie (FR); FILOCHE, Bruno, F-94000 Creteil (FR); MIGNANI, Serge, F-92290 Châtenay-Malabry (FR)
(86) Numéro de dépôt international: FR9801540
(87) Numéro de publication internationale: WO99003839

(56) Documents cités:
- WO-A-97/28813
- CHEMICAL ABSTRACTS, vol. 74, no. 25, 1971 Columbus, Ohio, US; abstract no. 142298e, page 636; XP002060324 & JP 07 029990 A (MEDICO-CHEM.RES.LAB.)
- CHEMICAL ABSTRACTS, vol. 109, no. 22, 1988 Columbus, Ohio, US; abstract no. 169743z, R.HARDT: "CURIE-POINT PYROLYSYS" page 655; XP002060298 & J.ANAL.APPL.PYROLYSIS, vol. 13, no. 3, 1988, pages 191-198, ENG
- CHEMICAL ABSTRACTS, vol. 105, no. 17, 1986 Columbus, Ohio, US; abstract no. 170812v, H.TSUCHIDA: "IDENTIFICATION OF NOVELPYRAZINES" page 607; XP002060325 & DEV.FOOD SCI., vol. 13, 1986, pages 85-94, JP
- CHEMICAL ABSTRACTS, vol. 73, no. 21, 1970 Columbus, Ohio, US; abstract no. 107839q, K.NISHIE ET AL.: "PHARMACOLOGY OF ALKYL AND HYDROXYALKYLPYRAZINES." page 216; XP002060214 & TXICOL. APPL. PHARMACOL., vol. 17, no. 1, 1970, pages 244-249, CALIF.

## Description

La présente invention concerne les médicaments contenant en tant que principe actif au moins un composé de formule : 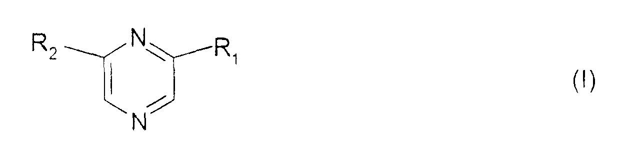 ou un de leurs stéréoisomères ou leurs sels avec un acide minéral ou organique, les nouveaux composés de formule (I) et leur préparation.

Dans la formule (I),
soit R₁ représente une chaîne -CH(Ra)-CHOH-CHOH-CH₂OH et R₂ représente une chaîne -CH₂-CHOH-CHOH-CH₂OH,
soit R₁ représente une chaîne -CHOH-CHF-CHOH-CH₂OH et R₂ représente une chaîne -CH₂-CHF-CHOH-CH₂OH,
soit R₁ représente une chaîne -CHOH-CHOH-CHOH-Rb et R₂ représente une chaîne -CH₂-CHOH-CHOH-Rb,
soit R₁ représente une chaîne -CH₂-CHOH-CHOH-CH₂OH et R₂ représente une chaîne -CH₂-CHOH-CHOH-CH₂OH,
soit R₁ et R₂ sont identiques et représentent chacun une chaîne - (CHOH)n-CH₂OH dans laquelle n est égal à 1, 2, 3 ou 4,
Ra représente un radical alcoxy (1-6 C en chaîne droite ou ramifiée) ou un atome de fluor,
Rb représente un radical carboxy, -CO-NH₂ ou -CH₂-NH₂.

Les composés de formule (I) comportant plusieurs carbones asymétriques, présentent des formes stéréoisomères. Ces différents stéréoisomères font partie de l'invention.

Le composé de formule : 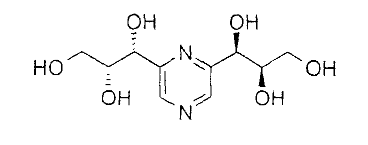 est décrit dans Nippon Shokuhin Kogyo Gakkaishi 37(2), 154 (1990), Agric. Biol. Chem. 44(5), 1189 (1980), Carbohydr. Res. 67(2), 549 (1978).

Le composé de formule : 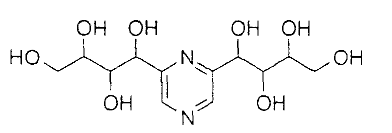 est décrit dans Dev. Food Sci. 13, 85 (1986).

Aucune activité pharmacologique n'est décrite pour ces dérivés.

Les autres composés de formule (I) sont nouveaux et en tant que tels font partie de la présente invention.

Les médicaments préférés sont ceux qui contiennent en tant que principe actif au moins un des composés suivants :
- 4-[6-(2,3,4-trihydroxy-butyl)-pyrazin-2-yl]-4-méthoxy-butane-1,2,3-triol,
- 4-[6-(2,3,4-trihydroxy-butyl)-pyrazin-2-yl]-4-fluoro-butane-1,2,3-triol,
- 1-[6-(2-fluoro-3,4-dihydroxy-butyl)-pyrazin-2-yl]-2-fluoro-butane-1,3,4-triol,
- 1-[6-(2,3-dihydroxy-4-amino-butyl)-pyrazin-2-yl]-4-amino-butane-1,2,3-triol,
- Acide 4-[6-(2,3-dihydroxy-4-carboxy-propyl)-pyrazin-2-yl]-2,3,4-trihydroxy-butanoïque,
- 4-[6-(2,3-dihydroxy-4-carbamoyl-propyl)-pyrazin-2-yl]-2,3,4-trihydroxy-butanamide,
- 4-[6-(2,3,4-trihydroxy-butyl)-pyrazin-2-yl]-butane-1,2,3-triol, 1-[6-(1,2-Dihydroxy-éthyl)-pyrazin-2-yl]-éthane-1,2-diol, 1-[6-(1,2,3- Trihydroxy-propyl)-pyrazin-2-yl]-propane-1,2,3-triol, 1-[6-(1,2,3,4-Tétrahydroxy-butyl)-pyrazin-2-yl]-butane-1,2,3,4-tétraol, 1-[6-(1,2,3,4,5-Pentahydroxy-pentyl)-pyrazin-2-yl]-pentane-1,2,3,4S,5-pentaol,
leurs stéréoisomères et leurs sels avec un acide minéral ou organique pharmaceutiquement acceptable.

Les médicaments particulièrement préférés sont ceux qui contiennent un composé choisi parmi les composés suivants :
- 4-[6-(2S,3R,4-trihydroxy-butyl)-pyrazin-2-yl]-4R-méthoxy-butane-1,2R,3S-triol,
- 4-[6-(2R,3R,4-trihydroxy-butyl)-pyrazin-2-yl]-4S-méthoxy-butane-1,2R,3R-triol,
- 4-[6-(2S,3R,4-trihydroxy-butyl)-pyrazin-2-yl]-4S-méthoxy-butane-1,2R,3S-triol,
- 4-[6-(2S,3R,4-trihydroxy-butyl)-pyrazin-2-yl]-4R-fluoro-butane-1,2R,3R-triol,
- 1-[6-(2S-fluoro-3R,4-dihydroxy-butyl)-pyrazin-2-yl]-2S-fluoro-butane-1R,3R,4-triol,
- 4-[6-(2R,3R,4-trihydroxy-butyl)-pyrazin-2-yl]-4S-fluoro-butane-1,2R,3R-triol,
- 1-[6-(2S,3R-dihydroxy-4-amino-butyl)-pyrazin-2-yl]-4-amino-butane-1R,2R,3R-triol,
- 1-[6-(2S,3S-dihydroxy-4-amino-butyl)-pyrazin-2-yl]-4-amino-butane-1R,2R,3S-triol,
- Acide 4-[6-(2S,3S-dihydroxy-4-carboxy-propyl)-pyrazin-2-yl]-2S,3S,4R-trihydroxy-butanoïque,
- Acide 4-[6-(2R,3S-dihydroxy-4-carboxy-propyl)-pyrazin-2-yl]-2S,3R,4R-trihydroxy-butanoïque,
- 4-[6-(2S,3S-dihydroxy-4-carbamoyl-propyl)-pyrazin-2-yl]-2S,3S,4R-trihydroxy-butanamide,
- 4-[6-(2S,3R,4-trihydroxy-butyl)-pyrazin-2-yl]-butane-1,2R,3S-triol,
- 4-[6-(2R,3R,4-trihydroxy-butyl)-pyrazin-2-yl]-butane-1,2R,3R-triol, 1-[6-(1R,2-Dihydroxy-éthyl)-pyrazin-2-yl]-éthane-1R,2-diol, 1-[6-(1S,2-Dihydroxy-éthyl)-pyrazin-2-yl]-éthane-1S,2-diol, 1-[6-(1R,2S,3- Trihydroxy-propyl)-pyrazin-2-yl]-propane-1R,2S,3-triol, 1-[6-(1S,2R,3-Trihydroxy-propyl)-pyrazin-2-yl]-propane-1S,2R,3-triol, 1-[6-(1S,2S,3-Trihydroxy-propyl)-pyrazin-2-yl]-propane-1S,2S,3-triol, 1-[6-(1R,2R,3R,4-Tétrahydroxy-butyl)-pyrazin-2-yl]-butane-1R,2R,3R,4-tétraol, 1-[6-(1R,2R,3S,4-Tétrahydroxy-butyl)-pyrazin-2-yl]-butane-1R,2R,3S,4-tétraol, 1-[6-(1R,2S,3R,4-Tétrahydroxy-butyl)-pyrazin-2-yl]-butane-1R,2S,3R,4-tétraol, 1-[6-( 1R,2S,3S,4-Tétrahydroxy-butyl)-pyrazin-2-yl]-butane-1R,2S,3S,4-tétraol, 1-[6-(1S,2R,3R,4-Tétrahydroxy-butyl)-pyrazin-2-yl]-butane-1S,2R,3R,4-tétraol, 1-[6-(1S,2R,3S,4-Tétrahydroxy-butyl)-pyrazin-2-yl]-butane-1S,2R,3S,4-tétraol, 1-[6-(1S,2S,3R,4-Tétrahydroxy-butyl)-pyrazin-2-yl]-butane-1S,2S,3R,4-tétraol, 1-[6-(1S,2S,3S,4-Tétrahydroxy-butyl)-pyrazin-2-yl]-butane-1S,2S,3S,4-tétraol, 1-[6-(1R,2R,3R,4S,5-Pentahydroxy-pentyl)-pyrazin-2-yl]-pentane-1R,2R,3R,4S,5-pentaol, 1-[6-(1R,2S,3S,4R,5-Pentahydroxy-pentyl)-pyrazin-2-yl]-pentane-1R,2S,3S,4R,5-pentaol, 1-[6-(1R,2S,3R,4S,5-Pentahydroxy-pentyl)-pyrazin-2-yl]-pentane-1R,2S,3R,4S,5-pentaol, 1-[6-(1R,2R,3R,4R,5-Pentahydroxy-pentyl)-pyrazin-2-yl]-pentane-1R,2R,3R,4R,5-pentaol, 1-[6-(1R,2S,3R,4R,5-Pentahydroxy-pentyl)-pyrazin-2-yl]-pentane-1R,2S,3R,4R,5-pentaol, 1-[6-(1S,2R,3R,4R,5-Pentahydroxy-pentyl)-pyrazin-2-yl]-pentane-1S,2R,3R,4R,5-pentaol, 1-[6-(1S,2R,3S,4R,5-Pentahydroxy-pentyl)-pyrazin-2-yl]-pentane-1S,2R,3S,4R,5-pentaol
et leurs sels avec un acide minéral ou organique pharmaceutiquement acceptable.

Les médicaments encore plus particulièrement préférés sont ceux qui contiennent un composé choisi parmi les composés suivants :
- 4-[6-(2S,3R,4-trihydroxy-butyl)-pyrazin-2-yl]-4R-méthoxy-butane-1,2R,3S-triol,
- 4-[6-(2R,3R,4-trihydroxy-butyl)-pyrazin-2-yl]-4S-méthoxy-butane-1,2R,3R-triol,
- 4-[6-(2S,3R,4-trihydroxy-butyl)-pyrazin-2-yl]-4S-méthoxy-butane-1,2R,3S-triol,
- 4-[6-(2S,3R,4-trihydroxy-butyl)-pyrazin-2-yl]-4R-fluoro-butane-1,2R,3R-triol,
- 1-[6-(2S-fluoro-3R,4-dihydroxy-butyl)-pyrazin-2-yl]-2S-fluoro-butane-1 R,3R,4-triol,
- 4-[6-(2R,3R,4-trihydroxy-butyl)-pyrazin-2-yl]-4S-fluoro-butane-1,2R,3R-triol,
- 1-[6-(2S,3R-dihydroxy-4-amino-butyl)-pyrazin-2-yl]-4-amino-butane-1 R,2R,3R-triol,
- 1-[6-(2S,3S-dihydroxy-4-amino-butyl)-pyrazin-2-yl]-4-amino-butane-1R,2R,3S-triol,
- Acide 4-[6-(2S,3S-dihydroxy-4-carboxy-propyl)-pyrazin-2-yl]-2S,3S,4R-trihydroxy-butanoïque,
- Acide 4-[6-(2R,3S-dihydroxy-4-carboxy-propyl)-pyrazin-2-yl]-2S,3R,4R-trihydroxy-butanoïque,
- 4-[6-(2S,3S-dihydroxy-4-carbamoyl-propyl)-pyrazin-2-yl]-2S,3S,4R-trihydroxy-butanamide,
- 4-[6-(2S,3R,4-trihydroxy-butyl)-pyrazin-2-yl]-butane-1,2R,3S-triol,
- 4-(6-(2R,3R,4-trihydroxy-butyl)-pyrazin-2-yl]-butane-1,2R,3R-triol,
et leurs sels avec un acide minéral ou organique.

Les composés de formule (I) peuvent être préparés par action de formiate d'ammonium sur un ou deux dérivés de formule :

OHC-CHOH-Rc (II)

dans laquelle Rc représente une chaîne -CH(Ra)-CHOH-CHOH-CH₂OH, - CHOH-CHF-CHOH-CH₂OH, -CHOH-CHOH-CHOH-Rb, CH₂-CHOH-CHOH-CH₂OH ou -(CHOH)n-CH₂OH dans lequel n est égal à 1, 2, 3 ou 4, Ra représente un radical alcoxy (1-6 C en chaîne droite ou ramifiée) ou un atome de fluor et Rb représente un radical carboxy, -CO-NH₂ ou -CH₂-NH₂ ou un de ses stéréoisomères.

Cette réaction s'effectue généralement en milieu aqueux, à une température comprise entre 20°C et 100°C.

Les dérivés de formule (II) et leurs stéréoisomères sont commercialisés ou peuvent être obtenus à partir :
a) d'aldoses commercialement disponibles :
   - par des réactions d'épimérisation par application ou adaptation des méthodes décrites dans Adv. Carbohydr. Chem., 13, 63, (1958) notamment en milieu basique au moyen d'une solution aqueuse diluée de soude (0,03 à 0.05%), à une température comprise entre 20 et 40°C,
   - par des réactions d'allongement de chaîne par application ou adaptation des méthodes décrites dans «The Carbohydrates», éditeurs: W. Pigman et D. Horton, Academic Press, New-York, Volume IA, 133 (1972) et notamment en formant la cyanhydrine de l'aldose de départ (par exemple par action du cyanure de sodium en solution aqueuse, à une température comprise entre 10 et 30°C et en présence de soude, à un pH voisin de 9) puis hydrolyse de la fonction nitrile ainsi formée en acide correspondant par application ou adaptation des méthodes décrites dans Organic Synthesis volume 1 page 436 et volume III page 85 (par exemple à l'aide d'acide chlorhydrique ou d'acide sulfurique concentré, en solution aqueuse, à une température comprise entre 20°C et la température de reflux du milieu réactionnel), puis réduction de la fonction acide carboxylique en aldéhyde correspondant par application ou adaptation des méthodes décrites dans J. Am. Chem. Soc. 71, 122 (1949) notamment à l'aide d'un borohydrure d'un métal alcalin (le borohydrure de sodium par exemple), en solution aqueuse à une température comprise entre 20°C et la température d'ébullition du milieu réactionnel,
   - par des réactions de racourcissement de chaînes par application ou adaptation des méthodes décrites dans «The Carbohydrates», éditeurs: W. Pigman et D. Horton, Academic Press, New-York, Volume IB, 1980, page 929 ou Chem. Ber., 83, 559 (1950) et notamment en transformant la fonction aldéhyde de l'aldose en hydroxylamine correspondant par application ou adaptation des méthodes décrites dans Organic Synthesis volume II page 314 (par exemple à l'aide de chlorhydrate d'hydroxylamine, en solution aqueuse et en présence d'une base telle que le carbonate de sodium à une température comprise entre 20 et 50°C), puis action du 3,4-dinitrofluorobenzène en présence de dioxyde de carbone et d'une base telle le d'hydrogénocarbonate de sodium en solution aqueuse et d'un alcool aliphatique (alcool isopropylique par exemple), à une température comprise entre 50 et 80°C,
b) d'alcools allyliques correspondants par application ou adaptation des méthodes décrites dans Science, 220, 949 (1983) et notamment à l'aide d'hydroperoxyde de terbutyle en présence d'un complexe de titane (IV) tel que le complexe isopropylate de titane (IV) et tartrate de dialkyle optiquement pur (le tartrate de diéthyle par exemple), suivi de l'action successive de thiophénolate de sodium, d'acide para-chloroperbenzoïque dans l'anhydride acétique et d'hydrure de diisopropylaluminium.

Les dérivés de formule (II) peuvent également être obtenus par application ou adaptation des méthodes décrites dans J. Am. Chem. Soc., 113(21), 8137 (1991 ), Chem. Pharm. Bull., 35(7), 2894 (1987), Carbohydr. Res., 154, 127 (1986), Sen'i Gakkaihi, 35(12) 525 (1979), Chem. Ber., 101(7), 2294 (1968), J. Carbohydr. Chem., 3(2) 219 (1984) et Tetrahedron, 40(12) 2233 (1984) et les brevets WO 9310137 et WO 89-US51089029.

Les différents stéréoisomères des composés de formule (I) sont obtenus à partir des stéréoisomères correspondants des intermédiaires (II). Parmi ces stéréoisomères, on utilise de préférence le 3-méthoxy-D-glucopyranose, le 3-fluoro-3-déoxy-D-glucose, le 4-fluoro-4-déoxy-D-glucose, le 6-amino-6-déoxy-D-glucose, l'acide D-glucuronique, l'acide D-galacturonique, le D-glucuronamide et le 3-déoxy-D-glucose.

Il est entendu pour l'homme du métier que, pour la mise en oeuvre des procédés selon l'invention décrits précédemment, il peut être nécessaire d'introduire des groupes protecteurs des fonctions amino, hydroxy et carboxy afin d'éviter des réactions secondaires. Ces groupes sont ceux qui permettent d'être éliminés sans toucher au reste de la molécule. Comme exemples de groupes protecteurs de la fonction amino on peut citer les carbamates de tert-butyle ou de méthyle qui peuvent être régénérées au moyen d'iodotriméthylsilane. Comme exemples de groupes protecteurs de la fonction hydroxy, on peut citer les trialkylsilyle (triéthylsilyle par exemple), benzyle. Comme groupes protecteurs des fonctions carboxy, on peut citer les esters (méthoxyméthylester, tétrahydropyranylester, benzylester par exemple), les oxazoles et les 2-alkyl-1,3-oxazolines. D'autres groupes protecteurs utilisables dans ces procédés sont également décrits par W. GREENE et coll., Protective Groups in Organic Synthesis, second edition, 1991, Jonh Wiley & Sons et P.J. KOCIENSKI, Protecting groups, éditeur Thieme Verlag (1994).

Les mélanges réactionnels obtenus par les divers procédés décrits précédemment sont traités suivant des méthodes classiques physiques (évaporation, extraction, distillation, chromatographie, cristallisation par exemple) ou chimiques (formation de sels par exemple).

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré.

Ces sels font également partie de l'invention.

Comme exemples de sels pharmaceutiquement acceptables, peuvent être cités les sels d'addition avec les acides minéraux ou organiques tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate

Les composés de formule (I) préférés sont ceux mentionnés précédemment comme principe actif des médicaments préférés à l'exception des produits connus.

Les exemples suivants illustrent l'invention

### EXEMPLE 1

A une solution de 2 g de 3-méthoxy-D-glucopyranose dans 3,4 cm³ d'eau distillée on ajoute 3,2 g de formiate d'ammonium. Le mélange réactionnel est chauffé à reflux sous agitation à une température d'environ 100°C pendant 2 h. Après refroidissement à une température d'environ 25°C, le mélange est dilué avec 25 cm³ d' acétate d'éthyle et décanté. La phase aqueuse est lavée avec 25 cm³ d'acétate d'éthyle, puis concentrée sous pression réduite (2,7 kPa) à une température voisine de 70°C. Le résidu est repris dans 100 cm³ d'éthanol absolu puis agité pendant 24 h. Le précipité ainsi obtenu est filtré sur verre fritté, lavé plusieurs fois avec de l'éthanol absolu et le filtrat est concentré sous pression réduite (2,7 kPa) à une température voisine de 45°C (opération répétée une fois). L'huile résiduelle est chromatographiée sur une colonne de 160 g de silice (0,02-0,05 mm) éluée avec un mélange eau/éthanol 1:199 en volumes à pression atmosphérique et en recueillant des fractions de 10 cm³. Les fractions contenant le produit attendu sont réunies et concentrées sous pression réduite (2,7 kPa) à une température voisine de 55°C. On obtient ainsi le 4-[6-(2S,3R,4-trihydroxy-butyl)-pyrazin-2-yl]-4R-méthoxy-butane-1,2R,3S-triol [Spectre de R.M.N. ¹H (400 MHz, (CD₃)₂SO d6, δ en ppm) : 2,73 et 3,09 (2 dd, respectivement J = 14 et 9,5 Hz et J = 14 et 3 Hz, 1H chacun : CH₂ 6α) ; 3,34 (s, 3H : OCH₃ en 2α) ; de 3,30 à 3,55 et de 3,55 à 3,70 (2 mts : les 7H correspondant aux : CH 2β - CH 2γ - CH₂O 2δ - CH 6γ et CH₂O 6δ ) ; 3,79 (mt, 1 H : CH 6β) ; de 4,00 à 5,00 ( plusieuurs mfs étalés : OH) ; 4,61 (s large, 1H : CH 2α) ; 8.39 (s, 1 H : =CH en 5) ; 8,42 (s,1 H : =CH en 3).

### EXEMPLE 2

A une solution de 10 g de L-gulose dans 28 cm³ d'eau distillée on ajoute 21 g de formiate d'ammonium. Le mélange réactionnel est chauffé à reflux sous agitation à une température d'environ 100°C pendant 30 minutes. Après refroidissement à une température d'environ 25°C, le mélange est concentré sous pression réduite (2,7 kPa) à une température voisine de 50°C. Le résidu est repris 5 fois dans l'éthanol puis reconcentré dans les mêmes conditions et enfin repris dans l'éthanol et agité pendant 3h. Le précipité ainsi obtenu est filtré sur verre fritté, lavé avec de l'éther diéthylique et essoré. Le filtrat est concentré sous pression réduite (2,7 kPa) à une température voisine de 50°C. Le résidu est chromatographié sur une colonne de 800 g de silice (0,02-0,05 mm) éluée avec un mélange eau/éthanol 1:19 en volumes à pression atmosphérique et en recueillant des fractions de 30 cm³. Les fractions contenant le produit attendu sont réunies et concentrées sous pression réduite (2,7 kPa) à une température voisine de 40°C. Le produit obtenu est recristallisé dans un mélange eau/ éthanol, puis séché sous pression réduite (2,7 kPa) à une température voisine de 25°C. On isole ainsi le 1-[6-(1R,2S,3S,4-tétrahydroxy-butyl)-pyrazin-2-yl]-butane-1R,2S,3S,4-tétraol.Les composés de formule (I) présentent des propriétés pharmacologiques intéressantes. Ce sont des hypoglycémiants.

L'activité hypoglycémiante des composés de formule (I) a été déterminée sur la réponse hyperglycémique à l'administration de glucose par la voie orale chez la souris normoglycémique, selon le protocole suivant :

Des souris Swiss albinos pesant entre 22 et 26 g sont laissées à jeûn pendant 2 heures. A la fin de cette période, la glycémie est mesurée et, immédiatement après, une dose de glucose (2 g/kg) est administrée par voie orale. Trente minutes plus tard, la glycémie est mesurée encore une fois. Les souris qui répondent par une hyperglycémie supérieure à 170 mg/dl sont sélectionnées et utilisées pour détecter l'activité hypoglycémiante des composés selon l'invention.

Les souris ainsi choisies sont réparties en groupes d'au moins 10 animaux. Des groupes distincts reçoivent une solution de 3 à 50 mg/kg du produit à tester dans un véhicule tel que l'eau ou un mélange de méthylcellulose/tween et eau ou du véhicule une fois par jour par tubage gastrique. Le traitement dure 4 jours. Au 4 ^{ème} jour, après le dernier traitement, les animaux reçoivent une dose de glucose (2 g/kg) et la glycémie est mesurée 20 à 40 minutes plus tard. Le pourcentage d'inhibition de la réponse hyperglycémique à l'administration de glucose est calculée par rapport à la réponse mesurée dans le groupe traité par le véhicule.

Dans ce test, les composés selon l'invention présentent un pourcentage d'inhibition de la glycémie supérieur ou égal à 10%.

Les composés de formule générale (I) selon l'invention présentent une faible toxicité. Leur DL50 est supérieure à 2000 mg/kg par voie orale chez la souris.

En thérapeutique humaine, ces produits sont utiles dans la prévention et le traitement du diabète et notamment du diabète de type II (NID diabète), du diabète de l'obèse, du diabète de la cinquantaine, du diabète métapléthorique, du diabète du sujet âgé et du diabète léger. Ils peuvent être utilisés en complément de l'insulinothérapie dans le diabète insulino dépendant où ils permettent de diminuer progressivement la dose d'insuline, le diabète instable, le diabète insulinorésistant, en complément des sulfamides hypoglycémiants quand ceux-ci ne déterminent pas de baisse suffisante de la glycémie. Ces produits peuvent être utilisés également dans les complications du diabète telles que les hyperlipémies, les troubles du métabolisme lipidique, les dyslipémies, l'obésité. Ils sont aussi utiles dans la prévention et le traitement des lésions d'athérosclérose et leurs complications (coronopathies, infarctus du myocarde, cardiomyopathies, évolution de ces trois complications vers l'insuffisance ventriculaire gauche, artériopathies diverses, artérites des membres inférieurs avec claudication et évolution vers les ulcères et la gangrène, insuffisance vasculaire cérébrale et ses complications, impuissance sexuelle d'origine vasculaire), la rétinopathie diabétique et de toutes ses manifestations (augmentation de la perméabilité capillaire, dilatation et thrombose capillaire, microanévrismes, shunt, artérioveineux, dilatation veineuse, hémorragies ponctiformes et maculaires, exudats, oedèmes maculaires, manifestations de la rétinopathie proliférante : néovaisseaux, cicatrices de rétinite proliférante, hémorragies du vitrée, décollement de la rétine), la cataracte diabétique, la neuropathie diabétique dans ses diverses formes (polyneuropathies périphériques et ses manifestations telles que paresthésies, hyperesthésies et douleurs, mononeuropathies, radiculopathies, neuropathies autonomes, amyotrophies diabétiques), les manifestations du pied diabétique (ulcères des extrémités inférieures et du pied), la néphropathie diabétique dans ses deux formes diffuse et nodulaire, l'athéromatose (élévation des HDL lipoproteïnes favorisant l'élimination du cholestrérol à partir des plaques d'athérome, baisse des LDL lipoproteïnes, baisse du rapport LDL/HDL, inhibition de l'oxydation des LDL, diminution de l'adhésivité plaquettaire), des hyperlipémies et des dyslipémies (hypercholestérolémies, hypertriglycéridémies, normalisation du taux des acides gras, normalisation de l'uricémie, normalisation des apoprotéïnes A et B), de la cataracte, de l'hypertension artérielle et ses conséquences.

Les médicaments selon l'invention sont constitués par un composé selon l'invention ou une combinaison de ces produits, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le pro-pylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée; elles sont généralement comprises entre 150 mg et 600 mg par jour par voie orale pour un adulte avec des doses unitaires allant de 50 mg à 200 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Produit actif 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Produit actif 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 50 mg de produit actif ayant la composition suivante :
- Produit actif 50 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

L'invention concerne également l'utilisation des composés de formule générale (I) pour la préparation de compositions pharmaceutiques utiles pour le traitement ou la prévention du diabète et des complications du diabète.

## Revendications

1. Médicaments contenant en tant que principe actif au moins un composé de formule : 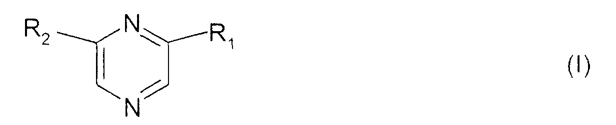 dans laquelle
soit R₁ représente une chaîne -CH(Ra)-CHOH-CHOH-CH₂OH et R₂ représente une chaîne -CH₂-CHOH-CHOH-CH₂OH,
soit R₁ représente une chaîne -CHOH-CHF-CHOH-CH₂OH et R₂ représente une chaîne -CH₂-CHF-CHOH-CH₂OH,
soit R₁ représente une chaîne -CHOH-CHOH-CHOH-Rb et R₂ représente une chaîne -CH₂-CHOH-CHOH-Rb,
soit R₁ représente une chaîne -CH₂-CHOH-CHOH-CH₂OH et R₂ représente une chaîne -CH₂-CHOH-CHOH-CH₂OH,
soit R₁ et R₂ sont identiques et représentent chacun une chaîne - (CHOH)n-CH₂OH dans laquelle n est égal à 1, 2, 3 ou 4,
Ra représente un radical alcoxy (1-6 C en chaîne droite ou ramifiée) ou un atome de fluor,
Rb représente un radical carboxy, -CO-NH₂ ou -CH₂-NH₂.
leurs stéréoisomères et leurs sels avec un acide minéral ou organique.

2. Médicaments contenant un composé de formule (I) selon la revendication 1 choisi parmi les composés suivants :
- 4-[6-(2,3,4-trihydroxy-butyl)-pyrazin-2-yl]-4-méthoxy-butane-1,2,3-triol,
- 4-[6-(2,3,4-trihydroxy-butyl)-pyrazin-2-yl]-4-fluoro-butane-1,2,3-triol,
- 1-[6-(2-fluoro-3,4-dihydroxy-butyl)-pyrazin-2-yl]-2-fluoro-butane-1,3,4-triol,
- 1-[6-(2,3-dihydroxy-4-amino-butyl)-pyrazin-2-yl]-4-amino-butane-1,2,3-4-triol,
- Acide 4-[6-(2,3-dihydroxy-4-carboxy-propyl)-pyrazin-2-yl]-2,3,4-trihydroxy-butanoïque,
- 4-[6-(2,3-dihydroxy-4-carbamoyl-propyl)-pyrazin-2-yl]-2,3,4-trihydroxy-butanamide,
- 4-[6-(2,3,4-trihydroxy-butyl)-pyrazin-2-yl]-butane-1,2,3-triol, 1-[6-(1,2-Dihydroxy-éthyl)-pyrazin-2-yl]-éthane-1,2-diol, 1-[6-(1,2,3-Trihydroxy-propyl)-pyrazin-2-yl]-propane-1,2,3-triol, 1-[6-(1,2,3,4-Tétrahydroxy-butyl)-pyrazin-2-yl]-butane-1,2,3,4-tétraol, 1-[6-(1,2,3,4,5-Pentahydroxy-pentyl)-pyrazin-2-yl]-pentane-1,2,3,4S,5-pentaol,
leurs stéréoisomères et leurs sels avec un acide minéral ou organique pharmaceutiquement acceptable.

3. Médicaments contenant en tant que principe actif au moins un composé de formule (I) selon la revendication 1 choisi parmi les composés suivants :
- 4-[6-(2S,3R,4-trihydroxy-butyl)-pyrazin-2-yl]-4R-méthoxy-butane-1,2R,3S-triol,
- 4-[6-(2R,3R,4-trihydroxy-butyl)-pyrazin-2-yl]-4S-méthoxy-butane-1,2R,3R-triol,
- 4-[6-(2S,3R,4-trihydroxy-butyl)-pyrazin-2-yl]-4S-méthoxy-butane-1,2R,3S-triol,
- 4-[6-(2S,3R,4-trihydroxy-butyl)-pyrazin-2-yl]-4R-fluoro-butane-1,2R,3R-triol,
- 1-[6-(2S-fluoro-3R,4-dihydroxy-butyl)-pyrazin-2-yl]-2S-fluoro-butane-1R,3R,4-triol,
- 4-[6-(2R,3R,4-trihydroxy-butyl)-pyrazin-2-yl]-4S-fluoro-butane-1,2R,3R-triol,
- 1-[6-(2S,3R-dihydroxy-4-amino-butyl)-pyrazin-2-yl]-4-amino-butane-1 R,2R,3R-triol,
- 1-[6-(2S,3S-dihydroxy-4-amino-butyl)-pyrazin-2-yl]-4-amino-butane-1R,2R,3S-triol,
- Acide 4-[6-(2S,3S-dihydroxy-4-carboxy-propyl)-pyrazin-2-yl]-2S,3S,4R-trihydroxy-butanoïque,
- Acide 4-[6-(2R,3S-dihydroxy-4-carboxy-propyl)-pyrazin-2-yl]-2S,3R,4R-trihydroxy-butanoïque,
- 4-[6-(2S,3S-dihydroxy-4-carbamoyl-propyl)-pyrazin-2-yl]-2S,3S,4R-trihydroxy-butanamide,
- 4-[6-(2S,3R,4-trihydroxy-butyl)-pyrazin-2-yl]-butane-1,2R,3S-triol,
- 4-[6-(2R,3R,4-trihydroxy-butyl)-pyrazin-2-yl]-butane-1,2R,3R-triol, 1-[6-(1R,2-Dihydroxy-éthyl)-pyrazin-2-yl]-éthane-1R,2-diol, 1-[6-(1S,2-Dihydroxy-éthyl)-pyrazin-2-yl]-éthane-1S,2-diol, 1-[6-(1R,2S,3-Trihydroxy-propyl)-pyrazin-2-yl]-propane-1R,2S,3-triol, 1-[6-(1S,2R,3- Trihydroxy-propyl)-pyrazin-2-yl]-propane-1S,2R,3-triol, 1-[6-(1S,2S,3-Trihydroxy-propyl)-pyrazin-2-yl]-propane-1S,2S,3-triol, 1-[6-(1R,2R,3R,4-Tétrahydroxy-butyl)-pyrazin-2-yl]-butane-1R,2R,3R,4-tétraol, 1-[6-(1R,2R,3S,4-Tétrahydroxy-butyl)-pyrazin-2-yl]-butane-1R,2R,3S,4-tétraol, 1-[6-(1R,2S,3R,4-Tétrahydroxy-butyl)-pyrazin-2-yl]-butane-1R,2S,3R,4-tétraol, 1-[6-(1R,2S,3S,4-Tétrahydroxy-butyl)-pyrazin-2-yl]-butane-1R,2S,3S,4-tétraol, 1-[6-(1S,2R,3R,4-Tétrahydroxy-butyl)-pyrazin-2-yl]-butane-1S,2R,3R,4-tétraol, 1-[6-(1S,2R,3S,4-Tétrahydroxy-butyl)-pyrazin-2-yl]-butane-1S,2R,3S,4-tétraol, 1-[6-(1S,2S,3R,4-Tétrahydroxy-butyl)-pyrazin-2-yl]-butane-1S,2S,3R,4-tétraol, 1-[6-(1S,2S,3S,4-Tétrahydroxy-butyl)-pyrazin-2-yl]-butane-1S,2S,3S,4-tétraol, 1-[6-(1R,2R,3R,4S,5-Pentahydroxy-pentyl)-pyrazin-2-yl]-pentane-1 R,2R,3R,4S,5-pentaol, 1-[6-(1R,2S,3S,4R,5-Pentahydroxy-pentyl)-pyrazin-2-yl]-pentane-1R,2S,3S,4R,5-pentaol, 1-[6-(1R,2S,3R,4S,5-Pentahydroxy-pentyl)-pyrazin-2-yl]-pentane-1R,2S,3R,4S,5-pentaol, 1-[6-(1R,2R,3R,4R,5-Pentahydroxy-pentyl)-pyrazin-2-yl]-pentane-1R,2R,3R,4R,5-pentaol, 1-[6-(1R,2S,3R,4R,5-Pentahydroxy-pentyl)-pyrazin-2-yl]-pentane-1R,2S,3R,4R,5-pentaol, 1-[6-(1S,2R,3R,4R,5-Pentahydroxy-pentyl)-pyrazin-2-yl]-pentane-1S,2R,3R,4R,5-pentaol, 1-[6-(1S,2R,3S,4R,5-Pentahydroxy-pentyl)-pyrazin-2-yl]-pentane-1S,2R,3S,4R,5-pentaol
et leurs sels avec un acide minéral ou organique pharmaceutiquement acceptable.

4. Médicaments contenant en tant que principe actif au moins un composé de formule (I) selon la revendication 1 choisi parmi les composés suivants :
- 4-[6-(2S,3R,4-trihydroxy-butyl)-pyrazin-2-yl]-4R-méthoxy-butane-1,2R,3S-triol,
- 4-[6-(2R,3R,4-trihydroxy-butyl)-pyrazin-2-yl]-4S-méthoxy-butane-1,2R,3R-triol,
- 4-[6-(2S,3R,4-trihydroxy-butyl)-pyrazin-2-yl]-4S-méthoxy-butane-1,2R,3S-triol,
- 4-[6-(2S,3R,4-trihydroxy-butyl)-pyrazin-2-yl]-4R-fluoro-butane-1,2R,3R-triol,
- 1-[6-(2S-fluoro-3R,4-dihydroxy-butyl)-pyrazin-2-yl]-2S-fluoro-butane-1R,3R,4-triol,
- 4-[6-(2R,3R,4-trihydroxy-butyl)-pyrazin-2-yl]-4S-fluoro-butane-1,2R,3R-triol,
- 1-[6-(2S,3R-dihydroxy-4-amino-butyl)-pyrazin-2-yl]-4-amino-butane-1R,2R,3R-triol,
- 1-[6-(2S,3S-dihydroxy-4-amino-butyl)-pyrazin-2-yl]-4-amino-butane-1R,2R,3S-triol,
- Acide 4-[6-(2S,3S-dihydroxy-4-carboxy-propyl)-pyrazin-2-yl]-2S,3S,4R-trihydroxy-butanoïque,
- Acide 4-[6-(2R,3S-dihydroxy-4-carboxy-propyl)-pyrazin-2-yl]-2S,3R,4R-trihydroxy-butanoïque,
- 4-[6-(2S,3S-dihydroxy-4-carbamoyl-propyl)-pyrazin-2-yl]-2S,3S,4R-trihydroxy-butanamide,
- 4-[6-(2S,3R,4-trihydroxy-butyl)-pyrazin-2-yl]-butane-1,2R,3S-triol,
- 4-[6-(2R,3R,4-trihydroxy-butyl)-pyrazin-2-yl]-butane-1,2R,3R-triol,
et leurs sels avec un acide minéral ou organique.

5. Composés de formule : 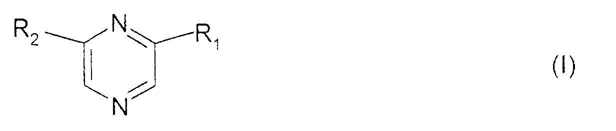 dans laquelle
soit R₁ représente une chaîne -CH(Ra)-CHOH-CHOH-CH₂OH et R₂ représente une chaîne -CH₂-CHOH-CHOH-CH₂OH,
soit R₁ représente une chaîne -CHOH-CHF-CHOH-CH₂OH et R₂ représente une chaîne -CH₂-CHF-CHOH-CH₂OH,
soit R₁ représente une chaîne -CHOH-CHOH-CHOH-Rb et R₂ représente une chaîne -CH₂-CHOH-CHOH-Rb,
soit R₁ représente une chaîne -CH₂-CHOH-CHOH-CH₂OH et R₂ représente une chaîne -CH₂-CHOH-CHOH-CH₂OH,
soit R₁ et R₂ sont identiques et représentent chacun une chaîne - (CHOH)n-CH₂OH dans laquelle n est égal à 1, 2, 3 ou 4,
Ra représente un radical alcoxy (1-6 C en chaîne droite ou ramifiée) ou un atome de fluor,
Rb représente un radical carboxy, -CO-NH₂ ou -CH₂-NH₂ à l'exception des composés de formule : 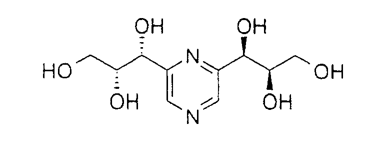 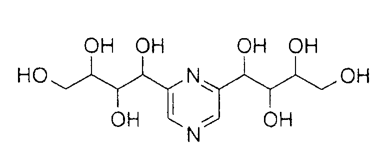
leurs stéréoisomères et leurs sels avec un acide minéral ou organique.

6. Procédé de préparation des composés de formule (I) selon la revendication 5 **caractérisé en ce que** l'on fait réagir le formiate d'ammonium sur un ou deux dérivés de formule :
OHC-CHOH-Rc (II)
dans laquelle Rc représente une chaîne -CH(Ra)-CHOH-CHOH-CH₂OH, - CHOH-CHF-CHOH-CH₂OH, -CHOH-CHOH-CHOH-Rb, CH₂-CHOH-CHOH-CH₂OH ou -(CHOH)n-CH2OH dans laquelle n est égal à 1, 2, 3 ou 4, Ra représente un radical alcoxy (1-6 C en chaîne droite ou ramifiée) ou un atome de fluor et Rb représente un radical carboxy, -CO-NH₂ ou -CH₂-NH₂ ou un de ses stéréoisomères, isole le produit et le transforme éventuellement en sel avec un acide minéral ou organique.

7. Utilisation d'un composé de formule (I) selon la revendication 1 ou un de ses stéréoisomères ou un de ses sels avec un acide minéral ou organique pour la préparation de compositions pharmaceutiques utiles pour le traitement ou la prévention du diabète et des complications du diabète.

## Patentansprüche

1. Medikamente, die als Wirkstoff enthalten wenigstens eine Verbindung der Formel: 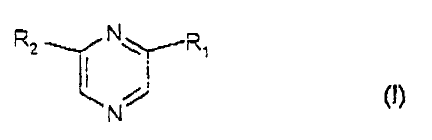 worin
entweder R₁ eine Kette -CH(Ra)-CHOH-CHOH-CH₂OH darstellt und R₂ eine Kette - CH₂-CHOH-CHOH-CH₂OH darstellt,
oder R₁ eine Kette -CHOH-CHF-CHOH-CH₂OH darstellt und R₂ eine Kette -CH₂CHF-CHOH-CH₂OH darstellt,
oder R₁ eine Kette -CHOH-CHOH-CHOH-Rb darstellt und R₂ eine Kette - CH₂-CHOH-CHOH-Rb darstellt,
oder R₁ eine Kette -CH₂-CHOH-CHOH-CH₂OH darstellt und R₂ eine Kette - CH₂-CHOH-CHOH-CH₂OH darstellt,
oder R₁ und R₂ gleich sind und jedes eine Kette -(CHOH)n-CH₂OH darstellt, worin n gleich 1, 2, 3 oder 4 ist,
Ra einen Alkoxyrest (1 - 6 C in gerader oder verzweigter Kette) oder ein Fluoratom darstellt,
Rb einen Carboxyrest, -CO-NH₂ oder -CH₂-NH₂ darstellt,
ihre Stereoisomere und ihre Salze mit einer mineralischen oder organischen Säure.

2. Medikamente, die eine Verbindung der Formel (I) gemäß Anspruch 1 enthalten, die ausgewählt ist unter den folgenden Verbindungen:
- 4-[6-(2,3,4-Trihydroxy-butyl)-pyrazin-2-yl]-4-methoxy-butan-1,2,3-triol,
- 4-[6-(2,3,4-Trihydroxy-butyl)-pyrazin-2-yl]-4-fluor-butan-1,2,3-triol,
- 1-[6-(2-Fluor-3,4-dihydroxy-butyl)-pyrazin-2-yl]-2-fluor-butan-1,3,4-triol,
- 1-[6-(2,3-Dihydroxy-4-amino-butyl)-pyrazin-2-yl]-4-amino-butan-1,2,3-triol,
- 4-[6-(2,3-Dihydroxy-3-carboxy-propyl)-pyrazin-2-yl]-2,3,4-trihydroxy-butansäure,
- 4-[6-(2,3-Dihydroxy-3-carbamoyl-propyl)-pyrazin-2-yl]-2,3,4-trihydroxy-butanamid,
- 4-[6-(2,3,4-Trihydroxy-butyl)-pyrazin-2-yl]-butan-1,2,3-triol,
- 1-[6-(1,2-Dihydroxy-ethyl)-pyrazin-2-yl]-ethan-1,2-diol,
- 1-[6-(1,2,3-Trihydroxy-propyl)-pyrazin-2-yl]-propan-1,2,3-triol,
- 1-[6-(1,2,3,4-Tetrahydroxy-butyl)-pyrazin-2-yl]-butan-1,2,3,4-tetraol,
- 1-[6-(1,2,3,4,5-Pentahydroxy-pentyl)-pyrazin-2-yl]-pentan-1,2,3,4S,5-pentaol,
ihren Stereoisomeren und ihren Salzen mit einer pharmazeutisch annehmbaren mineralischen oder organischen Säure.

3. Medikamente, die als Wirkstoff wenigstens eine Verbindung der Formel (I) gemäß Anspruch 1 enthalten, die ausgewählt ist unter den folgenden Verbindungen:
- 4-[6-(2S,3R,4-Trihydroxy-butyl)-pyrazin-2-yl]-4R-methoxy-butan-1,2R,3S-triol,
- 4-[6-(2R,3R,4-Trihydroxy-butyl)-pyrazin-2-yl]-4S-methoxy-butan-1,2R,3R-triol,
- 4-[6-(2S,3R,4-Trihydroxy-butyl)-pyrazin-2-yl]-4S-methoxy-butan-1,2R,3S-triol,
- 4-[6-(2S,3R,4-Trihydroxy-butyl)-pyrazin-2-yl]-4R-fluor-butan-1,2R,3R-triol,
- 1-[6-(2S-Fluor-3R,4-dihydroxy-butyl)-pyrazin-2-yl]-2S-fluor-butan-1R,3R,4-triol,
- 4-[6-(2R,3R,4-Trihydroxy-butyl)-pyrazin-2-yl]-4S-fluor-butan-1,2R,3R-triol,
- 1-[6-(2S,3R-Dihydroxy-4-amino-butyl)-pyrazin-2-yl]-4-amino-butan-1R,2R,3R-triol,
- 1-[6-(2S,3S-Dihydroxy-4-amino-butyl)-pyrazin-2-yl]-4-amino-butan-1R,2R,3S-triol,
- 4-[6-(2S,3S-Dihydroxy-3-carboxy-propyl)-pyrazin-2-yl]-2S,3S,4R-trihydroxy-butansäure,
- 4-[6-(2R,3S-Dihydroxy-3-carboxy-propyl)-pyrazin-2-yl]-2S,3R,4R-trihydroxy-butansäure,
- 4-[6-(2S,3S-Dihydroxy-3-carbamoyl-propyl)-pyrazin-2-yl]-2S,3S,4R-trihydroxy-butanamid,
- 4-[6-(2S,3R,4-Trihydroxy-butyl)-pyrazin-2-yl]-butan-1,2R,3S-triol,
- 4-[6-(2R,3R,4-Trihydroxy-butyl)-pyrazin-2-yl]-butan-1,2R,3R-triol,
- 1-[6-(1R,2-Dihydroxy-ethyl)-pyrazin-2-yl]-ethan-1R,2-diol,
- 1-[6-(1S,2-Dihydroxy-ethyl)-pyrazin-2-yl]-ethan-1S,2-diol,
- 1-[6-(1R,2S,3-Trihydroxy-propyl)-pyrazin-2-yl]-propan-1R,2S,3-triol,
- 1-[6-(1S,2R,3-Trihydroxy-propyl)-pyrazin-2-yl]-propan-1S,2R,3-triol,
- 1-[6-(1S,2S,3-Trihydroxy-propyl)-pyrazin-2-yl]-propan-1S,2S,3-triol,
- 1-[6-(1R,2R,3R,4-Tetrahydroxy-butyl)-pyrazin-2-yl]-butan-1R,2R,3R,4-tetraol,
- 1-[6-(1R,2R,3S,4-Tetrahydroxy-butyl)-pyrazin-2-yl]-butan-1R,2R,3S,4-tetraol,
- 1-[6-(1R,2S,3R,4-Tetrahydroxy-butyl)-pyrazin-2-yl]-butan-1R,2S,3R,4-tetraol,
- 1-[6-(1R,2S,3S,4-Tetrahydroxy-butyl)-pyrazin-2-yl]-butan-1R,2S,3S,4-tetraol,
- 1-[6-(1S,2R,3R,4-Tetrahydroxy-butyl)-pyrazin-2-yl]-butan-1S,2R,3R,4-tetraol,
- 1-[6-(1S,2R,3S,4-Tetrahydroxy-butyl)-pyrazin-2-yl]-butan-1S,2R,3S,4-tetraol,
- 1-[6-(1S,2S,3R,4-Tetrahydroxy-butyl)-pyrazin-2-yl]-butan-1S,2S,3R,4-tetraol,
- 1-[6-(1S,2S,3S,4-Tetrahydroxy-butyl)-pyrazin-2-yl]-butan-1S,2S,3S,4-tetraol,
- 1-[6-(1 R,2R,3R,4S,5-Pentahydroxy-pentyl)-pyrazin-2-yl]-pentan-1R,2R,3R,4S,5-pentaol,
- 1-[6-(1R,2S,3S,4R,5-Pentahydroxy-pentyl)-pyrazin-2-yl]-pentan-1R,2S,3S,4R,5-pentaol,
- 1-[6-(1R,2S,3R,4S,5-Pentahydroxy-pentyl)-pyrazin-2-yl]-pentan-1R,2S,3R,4S,5-pentaol,
- 1-[6-(1R,2R,3R,4R,5-Pentahydroxy-pentyl)-pyrazin-2-yl]-pentan-1R,2R,3R,4R,5-pentaol,
- 1-[6-(1 R,2S,3R,4R,5-Pentahydroxy-pentyl)-pyrazin-2-yl]-pentan-1R,2S,3R,4R,5-pentaol,
- 1-[6-(1S,2R,3R,4R,5-Pentahydroxy-pentyl)-pyrazin-2-yl]-pentan-1S,2R,3R,4R,5-pentaol,
- 1-[6-(1S,2R,3S,4R,5-Pentahydroxy-pentyl)-pyrazin-2-yl]-pentan-1S,2R,3S,4R,5-pentaol
und ihren Salzen mit einer pharmazeutisch annehmbaren mineralischen oder organischen Säure.

4. Medikamente, die als Wirkstoff wenigstens eine Verbindung der Formel (I) gemäß Anspruch 1 enthalten, die ausgewählt ist unter den folgenden Verbindungen:
- 4-[6-(2S,3R,4-Trihydroxy-butyl)-pyrazin-2-yl]-4R-methoxy-butan-1,2R,3S-triol,
- 4-[6-(2R,3R,4-Trihydroxy-butyl)-pyrazin-2-yl]-4S-methoxy-butan-1,2R,3R-triol,
- 4-[6-(2S,3R,4-Trihydroxy-butyl)-pyrazin-2-yl]-4S-methoxy-butan-1,2R,3S-triol,
- 4-[6-(2S,3R,4-Trihydroxy-butyl)-pyrazin-2-yl]-4R-fluor-butan-1,2R,3R-triol,
- 1-[6-(2S-Fluor-3R,4-dihydroxy-butyl)-pyrazin-2-yl]-2S-fluor-butan-1R,3R,4-triol,
- 4-[6-(2R,3R,4-Trihydroxy-butyl)-pyrazin-2-yl]-4S-fluor-butan-1,2R,3R-triol,
- 1-[6-(2S,3R-Dihydroxy-4-amino-butyl)-pyrazin-2-yl]-4-amino-butan-1R,2R,3R-triol,
- 1-[6-(2S,3S-Dihydroxy-4-amino-butyl)-pyrazin-2-yl]-4-amino-butan-1R,2R,3S-triol,
- 4-[6-(2S,3S-Dihydroxy-3-carboxy-propyl)-pyrazin-2-yl]-2S,3S,4R-trihydroxy-butansäure,
- 4-[6-(2R,3S-Dihydroxy-3-carboxy-propyl)-pyrazin-2-yl]-2S,3R,4R-trihydroxy-butansäure,
- 4-[6-(2S,3S-Dihydroxy-3-carbamoyl-propyl)-pyrazin-2-yl]-2S,3S,4R-trihydroxy-butanamid,
- 4-[6-(2S,3R,4-Trihydroxy-butyl)-pyrazin-2-yl]-butan-1,2R,3S-triol,
- 4-[6-(2R,3R,4-Trihydroxy-butyl)-pyrazin-2-yl]-butan-1,2R,3R-triol
und ihren Salzen mit einer mineralischen oder organischen Säure.

5. Verbindungen der Formel: 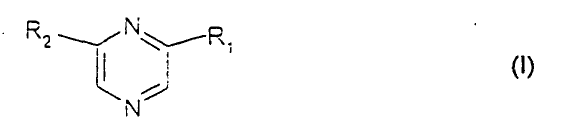 worin
entweder R₁ eine Kette -CH(Ra)-CHOH-CHOH-CH₂OH darstellt und R₂ eine Kette -CH₂-CHOH-CHOH-CH₂OH darstellt,
oder R₁ eine Kette -CHOH-CHF-CHOH-CH₂OH darstellt und R₂ eine Kette - CH₂-CHF-CHOH-CH₂OH darstellt,
oder R₁ eine Kette -CHOH-CHOH-CHOH-Rb darstellt und R₂ eine Kette - CH₂-CHOH-CHOH-Rb darstellt,
oder R₁ eine Kette -CH₂-CHOH-CHOH-CH₂OH darstellt und R₂ eine Kette - CH₂-CHOH-CHOH-CH₂OH darstellt,
oder R₁ und R₂ gleich sind und jedes eine Kette -(CHOH)n-CH₂OH darstellt, worin n gleich 1, 2, 3 oder 4 ist,
Ra einen Alkoxyrest (1 - 6 C in gerader oder verzweigter Kette) oder ein Fluoratom darstellt,
Rb einen Carboxyrest, -CO-NH₂ oder -CH₂-NH₂ darstellt mit Ausnahme der Verbindungen der Formel: 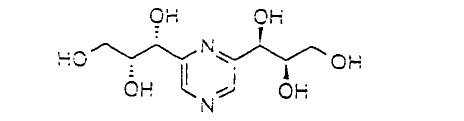 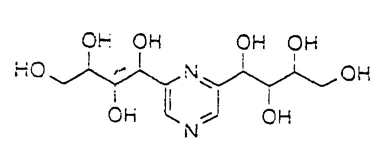
ihre Stereoisomere und ihre Salze mit einer mineralischen oder organischen Säure.

6. Verfahren zur Herstellung der Verbindungen der Formel (I) gemäß Anspruch 5, **dadurch gekennzeichnet, dass** man Ammoniumformiat mit einem oder zwei Derivaten der Formel:
OHC-CHOH-Rc (II)
worin Rc eine Kette -CH(Ra)-CHOH-CHOH-CH₂OH, -CHOH-CHF-CHOH-CH₂OH, - CHOH-CHOH-CHOH-Rb, -CH₂-CHOH-CHOH-CH₂OH oder -(CHOH)n-CH₂OH darstellt, worin n gleich 1, 2, 3 oder 4 ist, Ra einen Alkoxyrest (1 - 6 C in gerader oder verzweigter Kette) oder ein Fluoratom darstellt und Rb einen Carboxyrest, -CO-NH₂ oder -CH₂-NH₂ darstellt, oder einem seiner Stereoisomere umsetzt, das Produkt isoliert und es gegebenenfalls mit einer mineralischen oder organischen Säure in ein Salz umwandelt.

7. Verwendung einer Verbindung der Formel (I) gemäß Anspruch 1 oder von einem ihrer Stereoisomere oder einem ihrer Salze mit einer mineralischen oder organischen Säure für die Herstellung von pharmazeutischen Zusammensetzungen, die für die Behandlung oder die Vorbeugung des Diabetes und der Komplikationen des Diabetes verwendbar sind.

## Claims

1. Medicaments comprising, as active principle, at least one compound of formula: 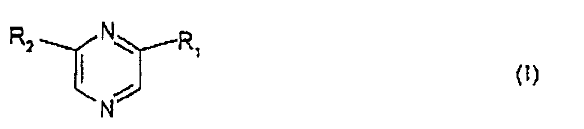 in which
either R₁ represents a -CH(Ra)-CHOH-CHOH-CH₂OH chain and R₂ represents a -CH₂-CHOH-CHOH-CH₂OH chain,
or R₁ represents a -CHOH-CHF-CHOH-CH₂OH chain and R₂ represents a -CH₂-CHF-CHOH-CH₂OH chain,
or R₁ represents a -CHOH-CHOH-CHOH-Rb chain and R₂ represents a -CH₂-CHOH-CHOH-Rb chain,
or R₁ represents a -CH₂-CHOH-CHOH-CH₂OH chain and R₂ represents a -CH₂-CHOH-CHOH-CH₂OH chain,
or R₁ and R₂ are identical and each represent a -(CHOH)ₙ-CH₂OH chain in which n is equal to 1, 2, 3 or 4,
Ra represents an alkoxy radical (1-6 C in a straight or branched chain) or a fluorine atom,
Rb represents a carboxyl, -CO-NH₂ or -CH₂-NH₂ radical, their stereoisomers and their salts with an inorganic or organic acid.

2. Medicaments comprising a compound of formula (I) according to claim 1 chosen from the following compounds:
- 4-[6-(2,3,4-Trihydroxybutyl)pyrazin-2-yl]-4-methoxybutane-1,2,3-triol,
- 4-[6-(2,3,4-Trihydroxybutyl)pyrazin-2-yl]-4-fluorobutane-1,2,3-triol,
- 1-[6-(2-Fluoro-3,4-dihydxoxybutyl)pyrazin-2-yl]-2-fluorobutane-1,3,4-triol,
- 1-[6-(2,3-Dihydroxy-4-aminobutyl)pyrazin-2-yl]-4-aminobutane-1,2,3-triol,
- 4-[6-(2,3-Dihydroxy-3-carboxypropyl)pyrazin-2-yl]-2,3,4-trihydroxybutanoic acid,
- 4-[6-(2,3-Dihydroxy-3-carbamoylpropyl)pyrazin-2-yl]-2,3,4-trihydroxybutariamide,
- 4-[6-(2,3,4-Trihydroxybutyl)pyrazin-2-yl]butane-1,2,3-triol, 1-[6-(1,2-Dihydroxyethyl)pyrazin-2-yl]ethane-1,2-diol, 1-[6-(1,2,3-Trihydroxypropyl)pyrazin-2-yl]propane-1,2,3-triol, 1-[6-(1,2,3,4-Tetrahydroxybutyl)pyrazin-2-yl]butane-1,2,3,4-tetraol, 1-[6-(1,2,3,4,5-Pentahydroxypentyl)pyrazin-2-yl]pentane-1,2,3,4S,5-pentaol,
their stereoisomers and their salts with a pharmaceutically acceptable inorganic or organic acid.

3. Medicaments comprising, as active principle, at least one compound of formula (I) according to claim 1 chosen from the following compounds:
- 4-[6-(2S,3R,4-Trihydroxybutyl)pyrazin-2-yl]-4R-methoxybutane-1,2R,3S-triol,
- 4-[6-(2R,3R,4-Trihydroxybutyl)pyrazin-2-yl]-4S-methoxybutane-1,2R,3R-triol,
- 4-[6-(2S,3R,4-Trihydroxybutyl)pyrazin-2-yl]-4S-methoxybutane-1,2R,3S-triol,
- 4-[6-(2S,3R,4-Trihydroxybutyl)pyrazin-2-yl]-4R-fluorobutane-1,2R,3R-triol,
- 1-[6-(2S-Fluoro-3R,4-dihydroxybutyl)pyrazin-2-yl]-2S-fluorobutane-1R,3R,4-triol,
- 4-[6-(2R,3R,4-Trihydroxybutyl)pyrazin-2-yl]-4S-fluorobutane-1,2R,3R-triol,
- 1-[6-(2S,3R-Dihydroxy-4-aminobutyl)pyrazin-2-yl]-4-aminobutane-1R,2R,3R-triol,
- 1-[6-(2S,3S-Dihydroxy-4-aminobutyl)pyrazin-2-yl]-4-aminobutane-1R,2R,3S-triol,
- 4-[6-(2S,3S-Dihydroxy-3-carboxypropyl)pyrazin-2-yl]-2S,3S,4R-trihydroxybutanoic acid,
- 4-[6-(2R,3S-Dihydroxy-3-carboxypropyl)pyrazin-2-yl]-2S,3R,4R-trihydroxybutanoic acid,
- 4-[6-(2S,3S-Dihydroxy-3-carbamoylpropyl)pyrazin-2-yl]-2S,3S,4R-trihydroxybutanamide,
- 4-[6-(2S,3R,4-Trihydroxybutyl)pyrazin-2-yl]butane-1,2R,3S-triol,
- 4-[6-(2R,3R,4-Trihydroxybutyl)pyrazin-2-yl]butane-1,2R,3R-triol, 1-[6-(1R,2-Dihydroxyethyl)pyrazin-2-yl]ethane-1R,2-diol, 1-[6-(1S,2-Dihydroxyethyl)pyrazin-2-yl]ethane-1S,2-diol, 1-[6-(1R,2S,3-Trihydroxypropyl)pyrazin-2-yl]propane-1R,2S,3-triol, 1-[6-(1S,2R,3-Trihydroxypropyl)pyrazin-2-yl]propane-1S,2R,3-triol, 1-[6-(1S,2S,3-Trihydroxypropyl)pyrazin-2-yl]propane-1S,2S,3-triol, 1-[6-(1R,2R,3R,4-Tetrahydroxybutyl)pyrazin-2-yl]butane-1R,2R,3R,4-tetraol, 1-[6-(1R,2R,3S,4-Tetrahydroxybutyl)pyrazin-2-yl]butane-1R,2R,3S,4-tetraol, 1-[6-(1R,2S,3R,4-Tetrahydroxybutyl)pyrazin-2-yl]butane-1R,2S,3R,4-tetraol, 1-[6-(1R,2S,3S,4-Tetrahydroxybutyl)pyrazin-2-yl]butane-1R,2S,3S,4-tetraol, 1-[6-(1S,2R,3R,4-Tetrahydroxybutyl)pyrazin-2-yl]butane-1S,2R,3R,4-tetraol, 1-[6-(1S,2R,3S,4-Tetrahydroxybutyl)pyrazin-2-yl]butane-1S,2R,3S,4-tetraol, 1-[6-(1S,2S,3R,4-Tetrahydroxybutyl)pyrazin-2-yl]butane-1S,2S,3R.4-tetraol, 1-[6-(1S,2S,3S,4-Tetrahydroxybutyl)pyrazin-2-yl]butane-1S,2S,3S,4-tetraol, 1-[6-(1R,2R,3R,4S,5-Pentahydroxypentyl)pyrazin-2-yl]pentane-1R,2R,3R,4S,5-pentaol, 1-[6-(1R,2S,3S,4R,5-Pentahydroxypentyl)pyrazin-2-yl]pentane-1R,2S,3S,4R,5-pentaol, 1-[6-(1R,2S,3R,4S,5-Pentahydroxypentyl)pyrazin-2-yl]pentane-1R,2S,3R,4S,5-pentaol, 1-[6-(1R,2R,3R,4R,5-Pentahydroxypentyl)pyrazin-2-yl]pentane-1R,2R,3R,4R,5-pentaol, 1-[6-(1R,2S,3R,4R,5-Pentahydroxypentyl)pyrazin-2-yl]pentane-1R,2S,3R,4R,5-pentaol, 1-[6-(1S,2R,3R,4R,5-Pentahydroxypentyl)pyrazin-2-yl]pentane-1S,2R,3R,4R,5-pentaol, 1-[6-(1S,2R,3S,4R,5-Pentahydroxypentyl)pyrazin-2-yl]pentane-1S,2R,3S,4R,5-pentaol,
and their salts with a pharmaceutically acceptable inorganic or organic acid.

4. Medicaments comprising, as active principle, at least one compound of formula (I) according to claim 1 chosen from the following compounds:
- 4-[6-(2S,3R,4-Trihydroxybutyl)pyrazin-2-yl]-4R-methoxybutane-1,2R,3S-triol,
- 4-[6-(2R,3R,4-Trihydroxybutyl)pyrazin-2-yl]-4S-methoxybutane-1,2R,3R-triol,
- 4-[6-(2S,3R,4-Trihydroxybutyl)pyrazin-2-yl]-4S-methoxybutane-1,2R,3S-triol,
- 4-[6-(2S,3R,4-Trihydroxybutyl)pyrazin-2-yl]-4R-fluorobutane-1,2R,3R-triol,
- 1-[6-(2S-Fluoro-3R,4-dihydroxybutyl)pyrazin-2-yl]-2S-fluorobutane-1R,3R,4-triol,
- 4-[6-(2R,3R,4-Trihydroxybutyl)pyrazin-2-yl]-4S-fluorobutane-1,2R,3R-triol,
- 1-[6-(2S,3R-Dihydroxy-4-aminobutyl)pyrazin-2-yl]-4-aminobutane-1R,2R,3R-triol,
- 1-[6-(2S,3S-Dihydroxy-4-aminobutyl)pyrazin-2-yl]-4-aminobutane-1R,2R,3S-triol,
- 4-[6-(2S,3S-Dihydroxy-3-carboxypropyl)pyrazin-2-yl]-2S,3S,4R-trihydroxybutanoic acid,
- 4-[6-(2R,3S-Dihydroxy-3-carboxypropyl)pyrazin-2-yl]-2S,3R,4R-trihydroxybutanoic acid,
- 4-[6-(2S,3S-Dihydroxy-3-carbamoylpropyl)pyrazin-2-yl]-2S,3S,4R-trihydroxybutanamide,
- 4-[6-(2S,3R,4-Trihydroxybutyl)pyrazin-2-yl]butane-1,2R,3S-triol,
- 4-[6-(2R,3R,4-Trihydroxybutyl)pyrazin-2-yl]butane-1,2R,3R-triol,
and their salts with an inorganic or organic acid.

5. Compounds of formula : 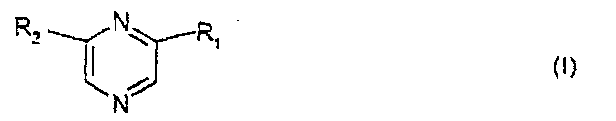 in which
either R₁ represents a -CH(Ra)-CHOH-CHOH-CH₂OH chain and R₂ represents a -CH₂-CHOH-CHOH-CH₂OH chain,
or R₁ represents a -CHOH-CHF-CHOH-CH₂OH chain and R₂ represents a -CH₂-CHF-CHOH-CH₂OH chain,
or R₁ represents a -CHOH-CHOH-CHOH-Rb chain and R₂ represents a -CH₂-CHOH-CHOH-Rb chain,
or R₁ represents a -CH₂-CHOH-CHOH-CH₂OH chain and R₂ represents a -CH₂-CHOH-CHOH-CH₂OH chain,
or R₁ and R₂ are identical and each represent a - (CHOH)ₙ-CH₂OH Chain in which n is equal to 1, 2, 3 or 4,
Ra represents an alkoxy radical (1-6 C in a straight or branched chain) or a fluorine atom,
Rb represents a carboxyl, -CO-NH₂ or -CH₂-NH₂ radical, with the exception of the compounds of formula: 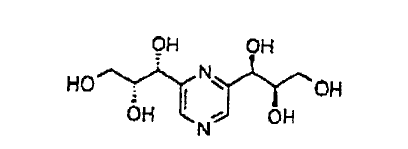 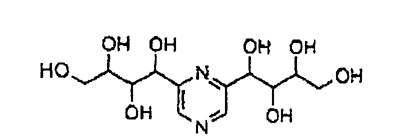
their stereoisomers and their salts with an inorganic or organic acid.

6. Process for the preparation of the compounds of formula (I) according to claim 5, **characterized in that** ammonium formate is reacted with one or two derivatives of formula:
OHC-CHOH-Rc (II)
in which Rc represents a -CH(Ra)-CHOH-CHOH-CH₂OH, -CHOH-CHF-CHOH-CH₂OH, -CHOH-CHOH-CHOH-Rb, CH₂-CHOH-CHOH-CH₂OH or -(CHOH)ₙ-CH₂OH chain in which n is equal to 1, 2, 3 or 4, Ra represents an alkoxy radical (1-6 C in a straight or branched chain) or a fluorine atom and Rb represents a carboxyl, -CO-NH₂ or -CH₂-NH₂ radical, or one of its stereoisomers, the product is isolated and is optionally converted to a salt with an inorganic or organic acid.

7. Use of a compound of formula (I) according to claim 1 or one of its stereoisomers or one of its salts with an inorganic or organic acid in the preparation of pharmaceutical compositions of use in the treatment or prevention of diabetes and complications of diabetes.
